# EUROPÄISCHE PATENTANMELDUNG

(11) **EP 1 316 297 A1**
(43) Veröffentlichungstag der Anmeldung: **04.06.2003**
(21) Anmeldenummer: 01811169.0
(22) Anmeldetag: 03.12.2001
(51) Int. Cl.: A61F 2/46

(54) **Vorrichtung zum Einbringen von Knochenzement in eine Spongiosa**

(71) Anmelder: Sulzer Orthopedics Ltd., 6340 Baar (CH)
(72) Erfinder: Grigoris, Peter Dr., Glasgow, Schotland G11 8DN (GB); Roberts, Paul Dr., Chepstow NP6 7BX (GB); Willi, Roland, 8413 Neftenbach (CH); O'Keane, Martin, 8400 Winterhur (CH)
(74) Vertreter: Manitz, Finsterwald & Partner

(57) **Zusammenfassung**

Vorgeschlagen wird eine Vorrichtung zum Einbringen von Knochenzement (1) in eine Spongiosa (2) eines präparierten Knochenendstücks (3). Die Vorrichtung umfasst eine Glocke (4) mit Hauptöffnung (5) und einen Hohlraum (6) zur Aufnahme des präparierten Knochenendstücks (3), sowie eine in den Hohlraum (6) der Glocke (4) einbringbare Saugeinrichtung (7), mit welcher ein einstellbarer Unterdruck innerhalb der Spongiosa (2) im Vergleich zum Hohlraum (6) der Glocke (4) erzeugbar ist. Die Glocke (4) weist im Bereich der Hauptöffnung (5) in Umfangsrichtung ein Dichtelement (8) zur Abdichtung des Hohlraums (6) der Glocke (4) an einem äusseren Umfang des Knochenendstücks (3) auf, sowie eine in den Hohlraum (6) einmündende Applikationsöffnung (9) zum Zuführen des Knochenzements (1).

## Beschreibung

Die Erfindung betrifft eine Vorrichtung zum Einbringen von Knochenzement in eine Spongiosa eines präparierten Knochenendstücks gemäss dem Oberbegriff des unabhängigen Anspruchs 1.

Bei der Implantation künstlicher Gelenke, zum Beispiel eines künstlichen Hüftgelenkes, ist es insbesondere bei jüngeren Patienten wichtig, die Resektion von Knochenmaterial auf ein absolut notwendiges Mass zu reduzieren, um möglichst viel vom Knochengewebe funktionstüchtig zu erhalten. So kann häufig die zerstörte Lauffläche eines Gelenkkopfes, beispielsweise eines Femurkopfes, durch eine künstliche Kappe wieder hergestellt werden. Durch eine solche Massnahme kann auf die Implantation eines künstlichen Hüftgelenkschaftes verzichtet und somit auch die vollständige Resektion des Femurkopfes vermieden werden. Der Gelenkkopf wird als Aufnahmestumpf auf eine Innenform der Kappe durch mechanische Vorbearbeitung angepasst. Dabei kann es aus verschiedenen Gründen sinnvoll sein, die Verankerung der Kappe auf dem Gelenkkopf mit Hilfe von Knochenzement vorzunehmen. So dient der Knochenzement zur Primärverankerung der Kappe auf dem präparierten Gelenkkopf und trägt zur mechanischen Stabilität der Verbindung bei.

Darüber hinaus ist es insbesondere von Vorteil, wenn der Knochenzement nicht nur die Oberfläche des Gelenkkopfes benetzt, sondern auch bis zu einer bestimmten Tiefe in die oberen Schichten der Spongiosa des Gelenkkopfes penetriert und damit zur mechanischen Stabilität der äusseren Bereiche der Spongiosa beiträgt. Die Penetration des Knochenzementes in die oberen Schichten der Spongiosa stellt bisher in sofern ein Problem dar, als dass dieser Vorgang nur ungenügend bzw. gar nicht kontrollierbar ist. So soll einerseits eine möglichst gleichmässige Penetration des Knochenzementes über den gesamten Oberflächenbereich des Gelenkkopfes erfolgen, andererseits soll auch die konkrete Dicke der Schicht kontrolliert eingestellt werden können. Eine ungleichmässige Penetration der Oberflächenschichten der Spongiosa beeinträchtigt deren mechanische Stabilität in den Randbereichen. Dringt dagegen der Knochenzement zu tief in die Spongiosa ein, können für den Knochen lebenswichtige Versorgungseinrichtungen, wie z.B. feine Blutgefässe, zerstört werden, was zum Absterben des Knochengewebes führen kann. Die EP 0 598 964 zeigt einen Körper zum Verteilen von Knochenzement für die Verankerung von Implantaten in einer vorbereiteten Knochenhöhlung. Diese Vorrichtung gestattet zwar ein gleichmässiges Ausspritzen des Zwischenraums zwischen Körper und Knochenaushöhlung, ist jedoch nicht geeignet zur Zementierung eines Knochenendstücks, wie beispielsweise eines Gelenkkopfes. Darüber hinaus gestattet es diese Vorrichtung nicht, die Penetration der oberen Schichten der vorbereiteten Knochenhöhlung gezielt zu steuern.

Die Aufgabe der Erfindung ist es daher, eine Vorrichtung zum kontrollierten Einbringen von Knochenzement in eine Spongiosa eines präparierten Knochenendstücks vorzuschlagen.

Die diese Aufgabe lösenden Gegenstände der Erfindung sind durch die Merkmale des unabhängigen Anspruchs 1 gekennzeichnet.

Die abhängigen Ansprüche beziehen sich auf besonders vorteilhafte Ausführungsformen der Erfindung.

Erfindungsgemäss wird also eine Vorrichtung zum Einbringen von Knochenzement in eine Spongiosa eines präparierten Knochenendstücks vorgeschlagen, welche eine Glocke mit Hauptöffnung und einen Hohlraum zur Aufnahme des präparierten Knochenendstücks, sowie eine in den Hohlraum der Glocke einbringbare Saugeinrichtung umfasst. Dabei ist mittels der Saugeinrichtung ein einstellbarer Unterdruck innerhalb der Spongiosa im Vergleich zum Hohlraum der Glocke erzeugbar, wobei die Glocke im Bereich der Hauptöffnung in Umfangsrichtung ein Dichtelement zur Abdichtung des Hohlraums der Glocke an einem äusseren Umfang des Knochenendstücks aufweist, sowie eine in den Hohlraum einmündende Applikationsöffnung zum Zuführen des Knochenzements.

Nachdem die Oberfläche des Knochenendstücks entsprechend vorbearbeitet worden ist, wird in der Spongiosa eine Bohrung präpariert, die geeignet ist, eine Saugeinrichtung zur Erzeugung eines Unterdrucks in der Spongiosa aufzunehmen. Dann wird die Glocke der erfindungsgemässen Vorrichtung so auf das präparierte Knochenendstück aufgesetzt, dass diese den gesamten Bereich der Oberfläche des Knochenendstücks umschliesst, der mit Knochenzement behandelt werden soll. Anschliessend wird die Saugeinrichtung in die zuvor in die Spongiosa eingebrachte Bohrung eingeführt. Dabei können die zuvor geschilderten Schritte selbstverständlich auch in einer anderen Reihenfolge durchgeführt werden.

Die Glocke weist im Bereich der Hauptöffnung in Umfangsrichtung ein Dichtelement auf, das den Hohlraum der Glocke an einem äusseren Umfang des Knochenendstückes nach aussen hin abdichtet. Das Dichtelement ist vorzugsweise als elastischer Ring ausgebildet, der mit zusätzlichen Einrichtungen ausgestattet sein kann, die die Abdichtwirkung erhöhen bzw. das Entfernen der Glocke erleichtern. Denkbar sind zum Beispiel einfache mechanische Einrichtungen wie ein Seilzug, der es erlaubt, das elastische Dichtelement an den äusseren Umfang des Knochenendstücks anzupressen. Bei einer anderen Variante könnte das Dichtelement beispielsweise als elastisch dehnbarer Schlauch ausgebildet sein, der von aussen mittels eines Fluids mit Druck beaufschlagbar ist und dadurch formgerecht an den äusseren Umfang des Knochenendstücks anpressbar ist. Darüber hinaus kann das Dichtelement lösbar mit der Glocke verbunden sein. Nach erfolgter Penetration der Spongiosa mit Knochenzement kann das Dichtelement, z.B. durch Entfernen einer in einen Umfang im Bereich zwischen Glocke und Dichtelement eingearbeiteten Schlinge von der Glocke gelöst werden, wodurch die Glocke vom Knochenendstück einfacher entfernbar ist und ein Abstreifen von Knochenzement durch das Dichtelement beim Abziehen der Glocke weitgehend vermieden werden kann. Selbstverständlich können Dichtelement und Glocke aber auch eine nicht-lösbare Einheit bilden.

Der für die Penetration der Spongiosa vorgesehene Knochenzement wird in einem Vorratsbehälter bereit gestellt, der mit einer Applikationsöffnung, z.B. mittels einer Verschraubung, verbunden werden kann. Der Vorratsbehälter besitzt eine Öffnung, durch die der Knochenzement in den Hohlraum zwischen Glocke und Knochenendstück eingbringbar ist.

Durch die Saugeinrichtung ist in der Spongiosa ein vorgebbarer Unterdruck einstellbar, so dass zunächst im wesentlichen die Luft, die sich im Hohlraum zwischen Glocke und Knochendstück befindet absaugbar ist. Der Knochenzement ist durch Beaufschlagung des Vorratsbehälters mit einem äusseren Druck im Hohlraum zwischen Glocke und Knochenendstück gleichmässig verteilbar. Durch den Unterdruck innerhalb der Spongiosa in Bezug auf den Hohlraum der Glocke dringt der Knochenzement kontrolliert durch die poröse Oberfläche des Knochenendstücks in die oberen Schichten der Spongiosa ein. Die Eindringtiefe ist über den vorgebbaren Unterdruck in der Spongiosa steuerbar.

Nach erfolgter Penetration der Spongiosa mit Knochenzement kann die Glocke vom Knochenendstück entfernt werden und eine Kappe als künstlicher Ersatz für die Gelenkfläche auf das Knochenendstück aufgesetzt werden.

Im folgenden wird die Erfindung anhand von bevorzugten Ausführungsbeispielen und anhand der schematischen Zeichnung näher erläutert. Es zeigen:

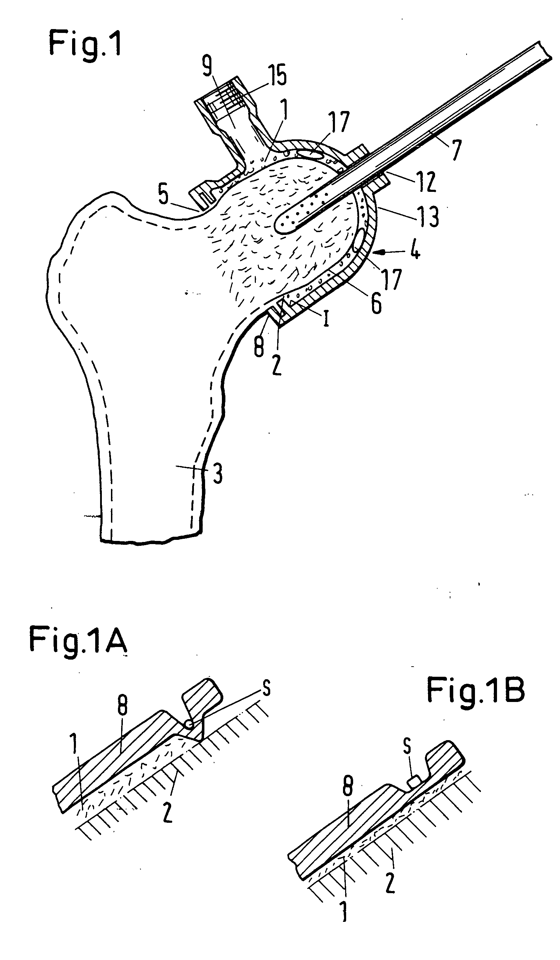

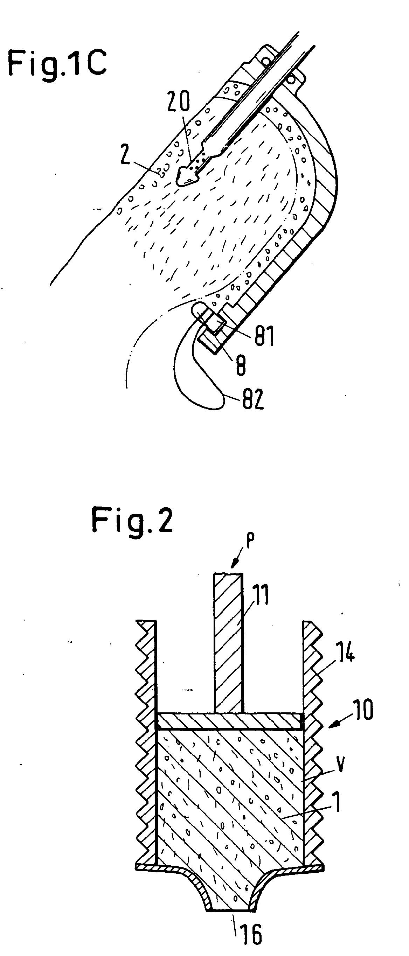

- Fig. 1:: einen Schnitt durch ein Ausführungsbeispiel der erfindungsgemässen Vorrichtung mit präpariertem Knochenendstück;
- Fig. 1a:: einen Schnitt durch ein Dichtelement mit Seilzug für eine Vorrichtung gemäss Fig. 1;
- Fig. 1 b:: wie Fig. 1 a, wobei der Seilzug nicht betätigt ist;
- Fig. 1c:: Ein Ausführungsbeispiel gemäss Fig. 1 mit Stützring;
- Fig. 2:: ein im Volumen verkleinerbarer Vorratsbehälter mit einer Stempeleinrichtung;
- Fig. 3:: wie Fig. 2 für ein Ausführungsbeispiel mit einem durch Druckbeaufschlagung mittels eines Fluids im Volumen vekleinerbaren Vorratsbehälter;

- Fig. 4:: ein weiteres Ausführungsbeispiel für einen im Volumen verkleinerbaren Vorratsbehälter gemäss Fig. 2 oder Fig. 3;
- Fig. 5:: eine schematische Darstellung einer Saugeinrichtung mit Absaugöffnungen und Ansaugkanal;
- Fig. 5a:: wie Fig.5, wobei sich die Bohrungen in einem Bereich mit reduziertem Durchmesser befinden;
- Fig. 6:: wie Fig. 1 für ein zweites Ausführungsbeispiel der erfindungsgemässen Vorrichtung;
- Fig. 7:: wie Fig 1 für ein drittes Ausführungsbeispiel einer erfindungsgemässen Vorrichtung.

Fig. 1 zeigt in einer schematischen Darstellung eine erfindungsgemässe Vorrichtung zum Einbringen von Knochenzement 1 in eine Spongiosa 2 eines präparierten Knochenendstücks 3. Die Vorrichtung besteht im wesentlichen aus einer Glocke 4 mit einer Hauptöffnung 5, welche Glocke ein präpariertes Knochenendstück 3, wie z.B. einen Femur-Kopf, aufnehmen kann. Der Innenraum der Glocke 4 erstreckt sich ausgehend von der Hauptöffnung 5 im wesentlichen als zylinderförmiger Hohlraum 6, wobei das der Hauptöffnung 5 gegenüberliegende Ende des zylinderförmigen Hohlraums 6 durch einen weitgehend sphärischen Abschnitt begrenzt ist, der eine Zusatzöffnung 12 aufweist. Durch die Zusatzöffnung 12 erstreckt sich eine Saugeinrichtung 7 in den Hohlraum der Glocke 4. Darüber hinaus besitzt die Glocke 4 noch eine Applikationsöffnung 9, die die Zuführung von Knochenzement 1 in den Hohlraum 6 erlaubt. An der Innenfläche I der Glocke 4 können sich Abstandselemente 17 in den Hohlraum 6 erstrecken, die einen einstellbaren Abstand zwischen Innenfläche I und der Oberfläche des Knochenendstücks 3 gewährleisten, um eine allseitige Penetration der Oberfläche des Knochenendstücks 3 mit Knochenzement sicherzustellen. Im Bereich der Hauptöffnung 5 weist die Glocke 4 in Umfangsrichtung ein Dichtelement 8 auf, das an einem äusseren Umfang des Knochenendstücks 3 den Hohlraum 6 bezüglich der Hauptöffnung 5 nach aussen abdichtet. Die Glocke 4 kann teilweise oder ganz aus einem Elastomer gefertigt sein, wobei zumindest das Dichtelement 8 elastische Eigenschaften aufweist, die es gestatten, dass das Dichtelement 8 über seinen gesamten Umfang dichtend am Knochenendstück 3 anliegt. Vorzugsweise ist die Glocke 4 zumindest teilweise aus einem transparenten Elastomer gefertigt, wodurch der Zustand der Benetzung mit Knochenzement beobachtbar ist. Dabei ist es durchaus denkbar, dass das Dichtelement 8 aus einem Elastomer gefertigt ist und die Glocke 4 aus einem anderen Material, wie zum Beispiel aus einem Metall, einer Metalllegierung oder einem anderen geeigneten Material besteht. Selbstverständlich kann die Glocke 4 auch aus dem gleichen Material wie das Dichtelement 8 gefertigt sein. Vorzugsweise ist das Dichtelement 8 in Umfangsrichtung aufweitbar, so dass es nach erfolgter Penetration des Knochenendstücks 3 mit Knochenzement 1 leicht entfernbar ist und eine ausreichende Benetzung der Oberfläche des Knochenendstücks 3 mit Knochenzement 1 erhalten bleibt. Darüber hinaus kann das Dichtelement 8 lösbar mit der Glocke 4 verbunden sein, so dass das Dichtelement 8 nach erfolgter Penetration der Spongiosa 2 mit Knochenzement 1 von der Glocke 4 lösbar ist, und damit die Glocke 4 wesentlich einfacher wieder von dem Knochenendstück 3 entfernbar ist. Zusätzlich kann das Dichtelement 8 pneumatische oder mechanische Zusatzeinrichtungen aufweisen, die die Dichtfunktion des Dichtelementes 8 unterstützen. Beispielsweise kann das Dichtelement 8 durch einen in Umfangsrichtung verlaufenden Seilzug S an einen äusseren Umfang des Knochenendstücks 3 angepresst werden. Die Fig. 1a und 1b zeigen schematische einen Schnitt durch ein Dichtelement 8 mit Seilzug S, wobei der Seilzug S in Umfangsrichtung der Hauptöffnung 5 der Glocke 4 entlang des Dichtelementes 8 verläuft. Erst nachdem der Seilzug S durch Zug betätigt wurde, bildet sich wie in Fig. 1a gezeigt, eine Wulst aus, die sich gegen die benachbarte Spongiosa 2 erstreckt und dadurch die Dichtwirkung erhöht. Ist dagegen wie in Fig. 1b gezeigt der Seilzug nicht betätigt, ist die Glocke 4 nach erfolgter Penetration mit Knochenzement 1 wieder entfernbar, so dass beim Abziehen der Glocke 4 eine genügend dicke Schicht Knochenzement 1 auf der Oberfläche der Spongiosa 2 verbleibt. Ebenso ist es vorstellbar, dass das Dichtelement 8 in Umfangsrichtung schlauchförmig ausgebildet ist, so dass das Dichtelemnet 8 von aussen durch ein Fluid mit einem vorgebbaren Druck beaufschlagt werden kann, wodurch das Dichtelement 8 an den äusseren Umfang des Knochenendstücks 3 angepressbar ist und dadurch die Dichtwirkung erhöht ist. Das Dichtelement 8 kann in einem weiteren Ausführungsbeispiel auch mittels eines entfernbaren Stützrings 81 an die Spongiosa angepresst werden. Dabei ist der Stützring 81, welcher in Umfangsrichtung mit einer lösbaren Verbindung zur Glocke 4 ausgestaltet ist, mittels einer Zugeinrichtung 82 gemeinsam mit dem Dichtelement 8 von der Glocke 4 entfernbar. Ein solcher Stützring 81 kann beispielsweise als ringförmige Wulst ausgebildet sein, die umlaufend eine Reissnut zur Glocke 4 aufweist und über die Zugeinrichtung 82, beginnend an einem radialen Schlitz, abreissbar ist.

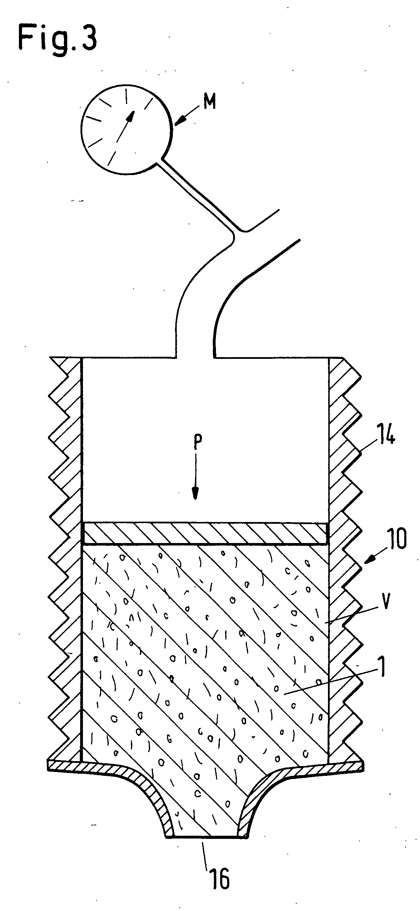

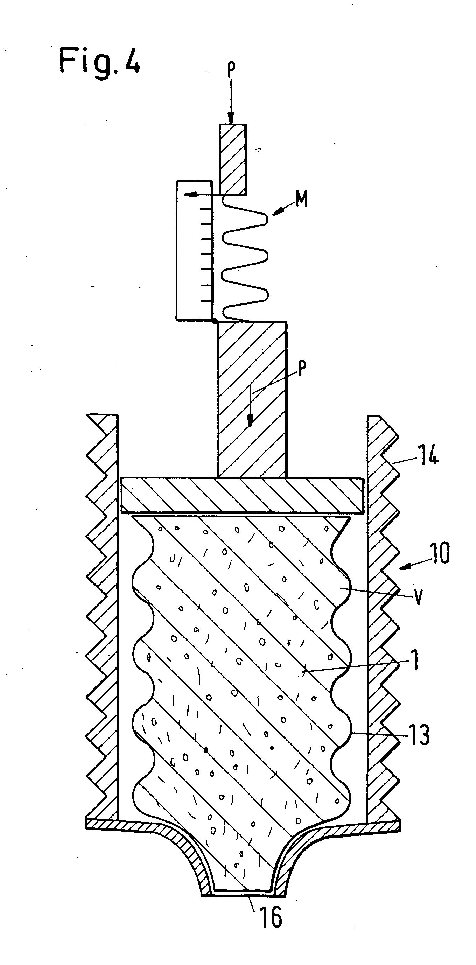

Die Fig. 2 bis 4 zeigen schematisch einen Vorratsbehälter 10, der zur Aufnahme einer vorgebbaren Menge von Knochenzement 1 geeignet ist und dazu dient, den Knochenzement 1 über die Applikationsöffnung 9 dem Hohlraum 6 der Glocke 4 zuzuführen. Der Vorratsbehälter 10 weist dabei geeignete Einrichtungen 14 auf, die es gestatten, den Vorratsbehälter 10 wirkfest mit der Applikationsöffnung 9 zu verbinden, die ihrerseits geeignete Einrichtungen 15 zur wirkfesten Verbindung mit dem Vorratsbehälter 10 aufweist. Beispielsweise kann der Vorratsbehälter 10 zur Verbindung mit der Applikationsöffnung 9 ein Aussengewinde 14 aufweisen, und die Applikationsöffnung 9 ein entsprechendes Innengewinde 15. Selbstverständlich sind auch andere Arten der Verbindung von Vorratsbehälter 10 und Applikationsöffnung 9 möglich. So kann die Applikationsöffnung 9 ein Aussengewinde 15 und der Vorratsbehälter 10 mit einem passenden Innengewinde 14 versehen sein. Auch z.B. BajonettVerschlüsse in den verschiedenen bekannten Varianten sind zur wirkfesten Verbindung des Vorratsbehälter 10 mit der Applikationsöffnung 9 denkbar, wie auch andere geeignete Befestigungsmittel. Der Vorratsbehälter kann von aussen durch einen vorgebbaren Druck P beaufschlagbar sein, wodurch ein Volumen V, das den Knochenzement 1 enthält, verkleinerbar ist. Durch Verkleinerung des Volumens V ist der Knochenzement 1 durch die Öffnung 16 dem Hohlraum 6 der Glocke 4 zuführbar und gleichmässig innerhalb der Glocke 4 über die Oberfläche des Knochenendstücks verteilbar. Die Beaufschlagung des Vorrates an Knochenzement 1 mit dem Druck P kann beispielsweise direkt mechanisch durch eine Stempeleinheit 11 oder durch eine geeignete pneumatische oder hydraulische Zusatzeinrichtung erfolgen, wobei der Druck P einstellbar und durch eine geeignete Einrichtung M messbar ist. Dabei kann, wie in Fig. 4 gezeigt, das den Knochenzement 1 aufnehmende Volumen V eventuell auch durch einen zusätzlichen Behälter B begrenzt sein, der in seinem Volumen verkleinerbar ausgestaltet ist.

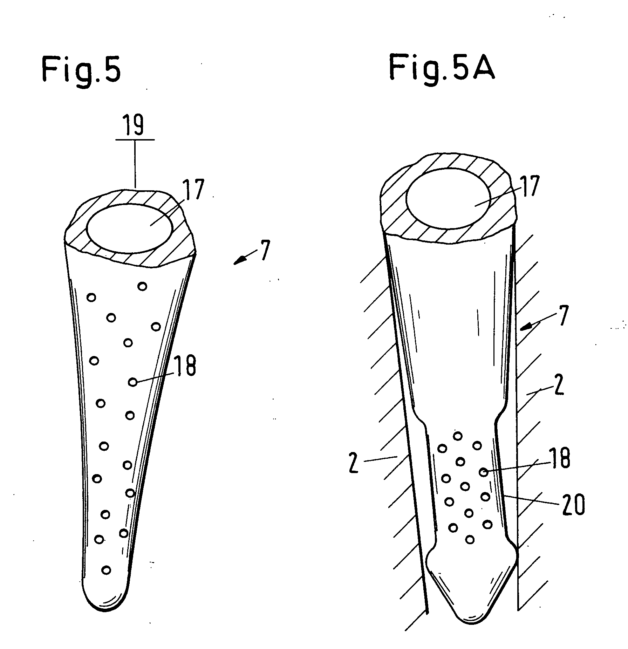

Ein wesentliches Element der erfindungsgemässen Vorrichtung ist die Saugeinrichtung 7. Wie in Fig. 5 schematisch dargestellt, umfasst die Saugeinrichtung 7 im wesentlichen einen inneren Ansaugkanal 17, wobei der Ansaugkanal 17 mindestens an einem Ende 19 der Saugeinrichtung 7 offen und an eine Einrichtung anschliessbar ist, mit der ein vorgebbarer Unterdruck im Inneren des Ansaugkanals 17 erzeugbar ist. Die Aussenwand der Saugeinrichtung 7 weist mindestens eine Durchbohrung 18 auf, die von aussen in den inneren Ansaugkanal 17 mündet. Die Saugeinrichtung 7 wird in die Bohrung 13 in die Spongiosa 2 eingebracht. Vorzugsweise weist der Innendurchmesser der Bohrung 13 in Bezug auf den Aussendurchmesser der Saugeinrichtung 7 ein gewisses Untermass auf. Ausserdem kann die Saugeinrichtung 7 in Richtung auf das Ende 19 sich konisch erweiternd ausgebildet sein. Dardurch kann einerseits gewährleistet werden, dass die Saugeinrichtung 7 ohne Spiel in der Bohrung 13 verankerbar ist und andererseits innerhalb der Bohrung 13 zwischen der Saugeinrichtung 7 und dem umgebenden Knochenmaterial der Spongiosa 2 eine gewisse Dichtwirkung erzielbar ist. In einem begrenzten Bereich 20 kann die Saugeinrichtung 7, wie in Fig. 1a bzw. Fig. 5a gezeigt, einen reduzierten Durchmesser aufweisen, so dass zwischen Spongiosa 2 und Saugeinrichtung 7 ein Hohlraum ausbildbar ist. Dadurch ist eine verbesserte Saugleistung erzielbar, weil die im Bereich 20 sich befindende Bohrung 18 nicht durch angrenzendes Knochenmaterial der Spongiosa 2 abgedeckt oder verstopft wird. Der durch die Saugeinrichtung 7 in der Spongiosa 2 vorgebbare Unterdruck hat zur Folge, dass ein äusserer Bereich der Spongiosa 2 bis in eine vorgebbare, von der Grösse des Unterdrucks abhängigen Tiefe von ca. 1 bis 5 mm gleichmässig mit Knochenzement ausfüllbar ist. Tiefere Schichten innerhalb der Spongiosa 2 bleiben weitgehend zementfrei, wodurch tieferliegendes Knochengewebe geschont wird.

Die in Fig. 1 gezeigte Variante eines bevorzugten Ausführungsbeispiels der erfindungsgemässen Vorrichtung mit Zusatzöffnung 12 erweist sich insbesondere dann als besonders vorteilhaft, wenn die künstliche Kappe, die auf den Gelenkkopf aufsetzbar ist, zur Primärverankerung im Knochenendstück 3 einen Verankerungsdorn aufweist. In diesem Fall dient die Bohrung 13 gleichzeitig zur Aufnahme des Verankerungsdorns der künstlichen Kappe. Es sind dann keinerlei Vorkehrungen notwendig, um die Bohrung 13 wieder zu verschliessen.

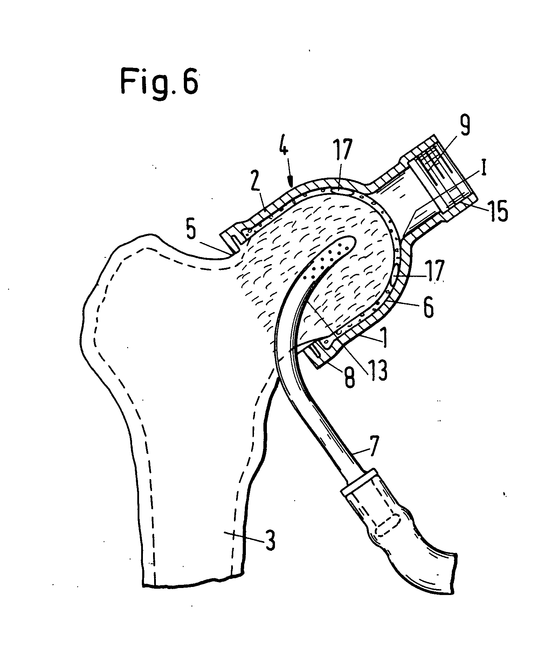

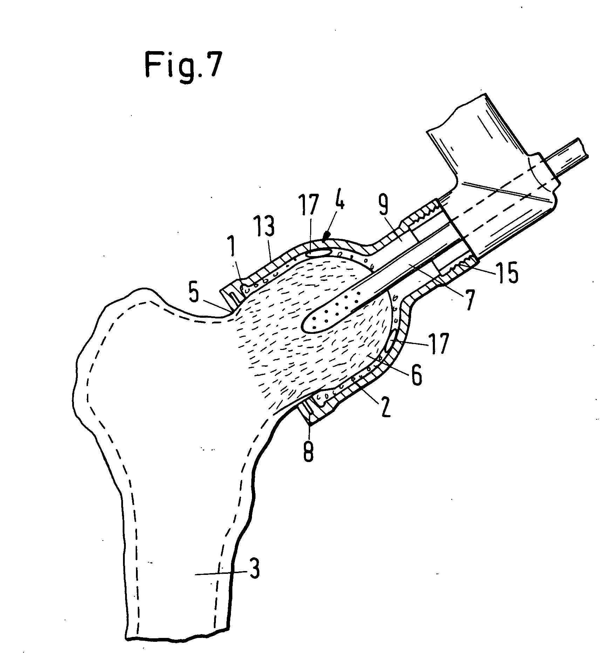

Die Fig. 6 und 7 zeigen weitere Ausführungsbeispiele der erfindungsgemässen Vorrichtung zum Einbringen von Knochenzement 1 in die Spongiosa 2. Fig. 6 zeigt ein zweites Ausführungsbeispiel, bei welchem sich die Saugeinrichtung 7 durch die Hauptöffnung 5 in die Glocke 4 erstreckt. Bei dieser Variante kann auf die Zusatzöffnung 12 ganz verzichtet werden.

Die Applikationsöffnung 9 kann dabei selbstverständlich auch anders an der Glocke 4 angeordnet sein als in Fig. 6 exemplarisch gezeigt. Fig. 7 zeigt ein drittes Ausführungsbeispiel, bei welchem sich die Saugeinrichtung 7 durch die Applikationsöffnung 9 in den Hohlraum 6 der Glocke 4 erstreckt. Auch bei dieser Variante kann auf die Zusatzöffnung 12 verzichtet werde und die Applikationsöffnung 12 kann auch hier selbstverständlich anders an der Glocke 4 angeordnet sein, als in Fig. 7 gezeigt.

Die Erfindung stellt also eine Vorrichtung bereit, mit welcher Knochenzement kontrollierbar in die Spongiosa eines präparierten Knochenendstücks einbringbar ist. Insbesondere bei jüngeren Patienten ist es ausserordentlich wichtig, zum Beispiel im Rahmen einer Wiederherstellung einer zerstörten Lauffläche eines Gelenkkopfes, möglichst viel vom Knochengewebe zu erhalten und, wenn irgend möglich, auf eine vollständige Resektion des natürlichen Gelenkkopfes zu verzichten. In diesen Fällen wird die zerstörte Lauffläche des Gelenkkopfes durch eine künstliche Kappe ersetzt, wobei die Primärverankerung derartiger künstlicher Kappen auf dem natürlichen Gelenkkopf durch Knochenzement unterstützt sein kann. Darüber hinaus soll der Knochenzement in der Regel jedoch nicht nur die Oberfläche benetzen, sondern auch bis zu einer bestimmten Tiefe in die oberen Schichten der Spongiosa des Gelenkkopfes penetrieren und damit zur mechanischen Stabilität der äusseren Bereiche der Spongiosa beitragen. Die erfindungsgemässe Vorrichtung ermöglicht es auf besonders einfache Weise, dass einerseits die gesamte Oberfläche des Knochenendstücks gleichmässig benetzbar ist und andererseits der Knochenzement kontrolliert in die oberen Schichten der Spongiosa in einer vorgebbaren Tiefe einbringbar ist. Einerseits ist dadurch eine erhöhte gleichmässige Stabilät der die künstliche Kappe tragenden Schichten der Spongiosa erreichbar. Andererseits ist weitgehend ausgeschlossen, dass der Knochenzement zu tief in die Spongiosa eindringt wodurch die Zerstörung von lebenswichtigen Versorgungseinrichtungen des Knochenmaterials verhindert wird. Es sei an dieser Stelle noch ausdrücklich darauf hingewiesen, dass selbstverständlich alle im Rahmen dieser Anmeldung beschriebenen Ausführungsbeispiele für eine Vorrichtung zur Einbringung von Knochenzement in eine Spongiosa auch ohne Saugeinrichtung 7 ausgestaltet sein können.

## Patentansprüche

1. Vorrichtung zum Einbringen von Knochenzement (1) in eine Spongiosa (2) eines präparierten Knochenendstücks (3) umfassend eine Glocke (4) mit Hauptöffnung (5) und einen Hohlraum (6) zur Aufnahme des präparierten Knochenendstücks (3), sowie eine in den Hohlraum (6) der Glocke (4) einbringbare Saugeinrichtung (7), mit welcher ein einstellbarer Unterdruck innerhalb der Spongiosa (2) im Vergleich zum Hohlraum (6) der Glocke (4) erzeugbar ist, wobei die Glocke (4) im Bereich der Hauptöffnung (5) in Umfangsrichtung ein Dichtelement (8) zur Abdichtung des Hohlraums (6) der Glocke (4) an einem äusseren Umfang des Knochenendstücks (3) aufweist, sowie eine in den Hohlraum (6) einmündende Applikationsöffnung (9) zum Zuführen des Knochenzements (1).

2. Vorrichtung nach Anspruch 1, bei welcher der Knochenzement (1) unter einem vorgebbaren Druck (P) aus einem im Volumen verkleinerbaren Vorratsbehälter (10) durch die Applikationsöffnung (9) in den Hohlraum (6) der Glocke (4) einbringbar ist.

3. Vorrichtung nach Anspruch 2, bei welcher der vorgegebbare Druck (P) auf einen im Volumen verkleinerbaren Vorratsbehälter (10) durch ein Fluid vermittelbar ist.

4. Vorrichtung nach Anspruch 2, bei welcher der vorgegebbare Druck (P) durch eine Stempeleinrichtung (11) auf den Knochenzement (1) vermittelbar ist.

5. Vorrichtung nach einem der Ansprüche 1 bis 4, bei welcher sich die Saugeinrichtung (7) durch die Applikationsöffnung (9) in die Glocke (4) erstreckt, um in der Spongiosa (2) einen Unterdruck zu erzeugen.

6. Vorrichtung nach einem der Ansprüche 1 bis 4, bei welcher sich die Saugeinrichtung (7) durch die Hauptöffnung (5) in die Glocke (4) erstreckt, um in der Spongiosa (2) einen Unterdruck zu erzeugen.

7. Vorrichtung nach einem der Ansprüche 1 bis 4, bei welcher sich die Saugeinrichtung (7) durch eine Zusatzöffnung (12) in die Glocke (4) erstreckt, um in der Spongiosa (2) einen Unterdruck zu erzeugen.

8. Vorrichtung nach einem der Ansprüche 1 bis 7, bei welcher die Abdichtfunktion des Dichtelementes (8) durch eine pneumatische Zusatzeinrichtung unterstützt ist.

9. Vorrichtung nach einem der Ansprüche 1 bis 7, bei welcher die Abdichtfunktion des Dichtelementes (8) durch eine mechanische Zusatzeinrichtung, beispielsweise durch einen Seilzug, unterstützt ist.

10. Vorrichtung nach einem der Ansprüche 1 bis 9, bei welcher das Dichtelement (8) durch einen abnehmbaren Stützring (81) gegen den Druck (P) gestützt und so bemessen ist, dass nach dem Einpressen des Knochenzements (1) und nach dem Entfernen des Stützrings (81) die Glocke (4) vom Knochenendstück (3) entfernbar ist.
